# EUROPEAN PATENT APPLICATION

(11) **EP 2 033 650 A1**
(43) Date of publication of application: **11.03.2009**
(21) Application number: 07767198.0
(22) Date of filing: 19.06.2007
(51) Int. Cl.: A61K 31/7048, A23L 1/30, A61K 8/60, A61K 8/97, A61K 36/00, A61K 36/28, A61P 3/06, A61P 3/10, A61P 9/10, A61P 9/12, A61P 11/00, A61P 11/06, A61P 13/08, A61P 15/00, A61P 17/00, A61P 17/02, A61P 17/04, A61P 17/06, A61P 17/10, A61P 19/02

(54) **INHIBITOR OF EXPRESSION OF NUCLEAR TRANSCRIPTION FACTOR AP-1, AND PHARMACEUTICAL PRODUCT AND PRODUCT USING THE INHIBITOR**

(30) Priority: 22.06.2006 JP 2006172997
(71) Applicant: Arkray, Inc., Kyoto-shi, Kyoto 601-8045 (JP)
(72) Inventor: YAGI, Masayuki, Minami-ku, Kyoto-shi, Kyoto 601-8045 (JP); MATSUURA, Nobuyasu, Okayama 701-1145 (JP)
(74) Representative: Behnisch, Werner
(86) International application number: PCT/JP2007/062340
(87) International publication number: WO 2007/148697

(57) **Abstract**

An expression inhibitor of a nuclear transcription factor AP-1 is provided that is excellent in safety and activity of inhibiting the expression of a nuclear transcription factor AP-1. The AP-1 expression inhibitor of the present invention is **characterized by** containing chamaemeloside. In the present invention, chamaemeloside may be contained as an extract of Roman chamomile or German chamomile. The chamaemeloside is contained in Roman chamomile or German chamomile. Conventionally, Roman chamomile and German chamomile have been used as cosmetic materials and herb teas and have no problems in safety. An extract of Roman chamomile or German chamomile and chamaemeloside inhibit the expression of the AP-1 at the gene level (see FIG. 2) and therefore are excellent in an inhibition effect as compared to a conventional inhibitor that inhibits binding of the AP-1 to DNA.

## Description

### Technical Field

The present invention relates to an expression inhibitor of a nuclear transcription factor AP-1 and pharmaceuticals and products using the same.

### Background Art

Cytokines such as interleukins and TNFs (tumor necrosis factors) are involved in onset of many diseases including cancer, arteriosclerosis, hypertension and diabetes. Cytokines are secreted in response to stimulation from outside and serve to induce various defense responses.

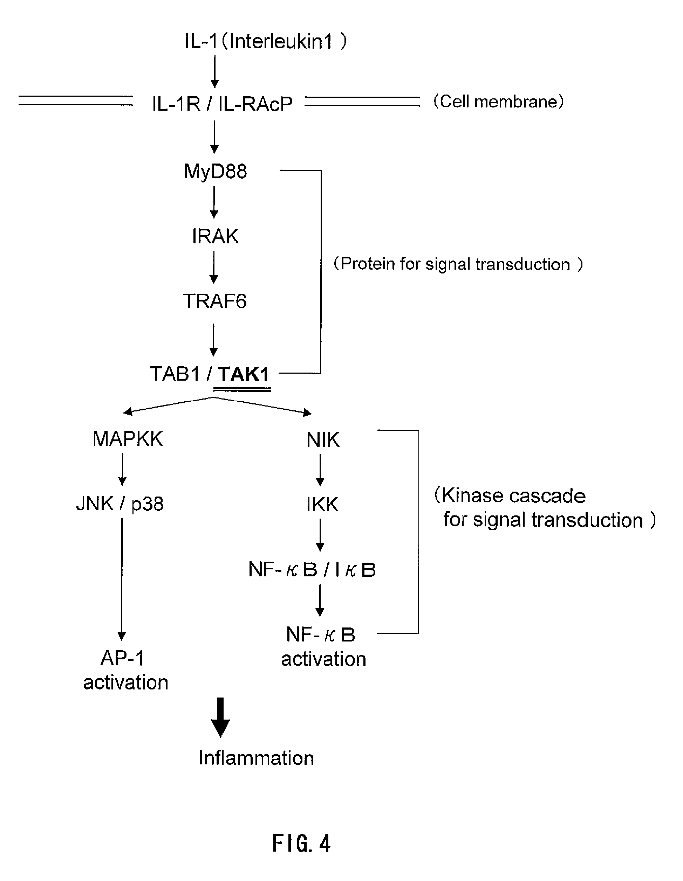

A response in which interleukin-1 (IL-1) is involved is explained with reference to FIG.4 as an example. As shown in the FIG. 4, when IL-1 binds to IL-1 receptor (IL-1R), a receptor complex is formed that consists of 4 proteins, namely IL-1R, IL-1R-AcP (IL-RAcP), MyD88, and IRAK. Thereafter, IRAK separates from the receptor complex within a few minutes and binds to an adaptor protein called TRAF6. Then TRAF6 binds to TAK1 and TAB1 to form a TAB1/TAK1 complex and thereby is activated. TAK1 is one of the kinases belonging to MAP kinase kinase (MAPKKK), and TAB1 is an activating factor of TAK1. The TAB1/TAK1 complex phosphorylates to activate both MAP kinase (MAPKK) and NIK (NF-κB inducing kinase), so that two downstream kinase cascades are activated. One of the kinase cascades activates a nuclear transcription factor AP-1 (hereinafter, referred to as "AP-1"). AP-1 is a transcriptional regulator belonging to the leucine zipper family, remains always bound to DNA, and is activated by phosphorylation. This phosphorylation is accomplished with JNK (c-Jun N-terminal kinase), which is a member of MAPKK, and p38. The other kinase cascade activates NF-κB (nuclear factor kappa B). NF-κB does not bind to DNA, generally is present in the cytoplasm while forming a complex with IκB (inhibitor-κB) protein, and is activated by degradation (separation) as a result of phosphorylation of IκB. This phosphorylation is accomplished with IKK and NIK. Activation of a nuclear transcription factor AP-1 and NF-κB induces an inflammatory response, so that, for example, diseases such as cancer, arteriosclerosis, hypertension, and diabetes may be caused.

As described above, AP-1 binds to DNA and a known DNA domain to which AP-1 binds is, for example, a specific TRE sequence (TGACTCA) or CRE sequence (TGACGTCA). Furthermore, for example, genes of cytokines such as interleukin-6 (IL-6) and TNF that play important roles in joint inflammation, and genes of metalloproteases such as collagenase and stromelysin that cause joint destruction each have the aforementioned specific sequence in the promoter region thereof. Therefore AP-1 binds to these DNAs to promote transcription of the aforementioned genes. Consequently, AP-1 has attracted attention as a potential drug target in relation to inflammatory diseases of connective tissues, such as rheumatoid arthritis. Theanine contained in natural products (Patent Document 1), a retinoid compound (Patent Document 2), an oligonucleotide (Patent Document 3), and a secreted material from Actinomyces (Patent Document 4) have been reported as specific examples of a substance that inhibits the activity of AP-1 by inhibiting the binding of AP-1 to DNA.
[Patent Document 1] JP 2003-55213 A
[Patent Document 2] JP 11(1999)-504328 A
[Patent Document 3] JP 10(1998)-36272 A
[Patent Document 4] JP 10(1998)-130201 A

### Disclosure of Invention

### Problem to be Solved by the Invention

Among the conventional substances that inhibit the activity of AP-1, those synthesized chemically have a problem in safety. Those derived from natural products are insufficient in their efficacy even though they are safe.

Therefore an object of the present invention is to provide an AP-1 inhibitor that is excellent in safety and in activity to inhibit AP-1.

### Means for Solving the Problem

In order to achieve the aforementioned object, an expression inhibitor of a nuclear transcription factor AP-1 of the present invention contains chamaemeloside.

A pharmaceutical of the present invention is a pharmaceutical for prevention or treatment of a disease in which a nuclear transcription factor AP-1 is involved or for wound healing, wherein the pharmaceutical contains an expression inhibitor of a nuclear transcription factor AP-1 of the present invention.

A product of the present invention is a product for prevention or relief of a disease in which a nuclear transcription factor AP-1 is involved or for acceleration of wound healing, wherein the product contains an expression inhibitor of a nuclear transcription factor AP-1 of the present invention.

An expression inhibition method of the present invention is a method of inhibiting expression of a nuclear transcription factor AP-1, wherein the method includes a step of administering chamaemeloside.

The method of the present invention is a method for preventing or treating a disease in which a nuclear transcription factor AP-1 is involved or for healing a wound, wherein the method includes a step of administering chamaemeloside.

Use of chamaemeloside of the present invention is use thereof for inhibiting expression of a nuclear transcription factor AP-1, or for preventing or treating a disease in which a nuclear transcription factor AP-1 is involved or for healing a wound.

### Effect of the Invention

In order to achieve the aforementioned object, the inventors have conducted a series of studies on materials, mainly natural products, which inhibit the activity of AP-1. As a result, they found that chamaemeloside contained in Roman chamomile or German chamomile, which was a type of Compositae plant, inhibited the expression of AP-1, and thereby achieved the present invention. Roman chamomile or German chamomile is being used as, for example, raw materials for foods such as herb teas or cosmetics, and there are no concerns about their safety. Since chamaemeloside inhibits the expression of AP-1, it can inhibit AP-1 activity more effectively as compared to the case of inhibiting the AP-1 activity through inhibition of binding of expressed AP-1 to DNA as in a conventional manner. In addition, chamaemeloside has not only the activity to inhibit AP-1 expression but also the activity to inhibit NF-κB and the activity to inhibit the Maillard reaction. Therefore, these activities work in synergy with the activity to inhibit AP-1 expression and produce remarkable effects on various diseases including inflammatory diseases.

### Brief Description of Drawings

FIG.1 is a diagram showing a chromatogram obtained by HPLC of an extract of Roman chamomile in an example of the present invention.
FIG.2 is a graph showing the effect of inhibiting the expression of a nuclear transcription factor AP-1 that is provided by chamaemeloside in the aforementioned example.
FIG.3 is a graph showing the effect of inhibiting the expression of NF-κB that is provided by an extract of Roman chamomile and chamaemeloside in Reference Example.
FIG.4 is a chart showing the relationships between interleukin-1 and a nuclear transcription factor AP-1 as well as NF-κB.
Fig.5 shows chromatograms obtained by HPLC in an example of the present invention; (a) is a chromatogram obtained by HPLC of an extract of German chamomile, and (b) is a chromatogram obtained by HPLC of chamaemeloside derived from Roman chamomile.

### Best Mode for Carrying Out the Invention

In the present invention, expression inhibition of the aforementioned AP-1 is at least one of expression inhibition at the gene level and expression inhibition at the protein level. The expression inhibition at the gene level involves inhibition of transcription to mRNA. The expression inhibition at the protein level involves inhibition in translation, and when modification is made after translation, it involves inhibition of the modification.

The AP-1 expression can be inhibited, for example, at the gene level or at the protein level, through, for example, administration or intake of the AP-1 expression inhibitor of the present invention. The AP-1 expression inhibitor of the present invention is not limited but it is preferable that the inhibitor be administered to or taken by, for example, humans and mammals other than humans.

Examples of chamaemeloside in the present invention include chamaemeloside derived from Roman chamomile (*Anthemis nobilis*) and chamaemeloside derived from German chamomile (*Matricaia* (*Mecutita*). The chamaemeloside derived from Roman chamomile may be derived from any part of the aforementioned Roman chamomile. For example, it may be derived from the whole plant body (herb), flower, stem, leaf, branch, branch with leaf, rhizome, root bark, root, or seed of Roman chamomile. Especially, chamaemeloside derived from the whole plant body and flower is preferable. Chamaemeloside derived from German chamomile may be derived from any part of the aforementioned German chamomile. For example, it may be derived from the whole plant body (herb), flower, stem, leave, branch, branch with leaf, rhizome, root bark, root, or seed of German chamomile. Especially, chamaemeloside derived from the whole plant body and flower is preferable.

The expression inhibitor of the present invention, as described above, is required only to contain chamaemeloside, and thus, for example, the form of chamaemeloside contained in the inhibitor is not limited. That is, the expression inhibitor of the present invention may contain, for example, purified chamaemeloside or the whole or a part of Roman chamomile or German chamomile plant body to contain chamaemeloside. An example in the former case is chamaemeloside obtained by purification of an extract of Roman chamomile or German chamomile. Chamaemeloside obtained by purification of the extract may be a purified product of single ingredient or a partially purified product. An example in the latter case is an extract from the whole or a part of Roman chamomile or German chamomile plant body (for example, in an unpurified crude state) or a crushed product of the whole or a part of Roman chamomile or German chamomile plant body. Both or either one of Roman chamomile and German chamomile may be contained. Furthermore, the aforementioned chamaemeloside may be, for example, a synthetic product or a commercially available product.

The chemical name of the aforementioned chamaemeloside is apigenin 7-glucoside-6"-(3"'-hydroxy-3"'-methyl-glutarate) represented by the chemical formula shown below. Chamaemeloside of the present invention may be a salt of chamaemeloside (for example, Na salt or K salt).

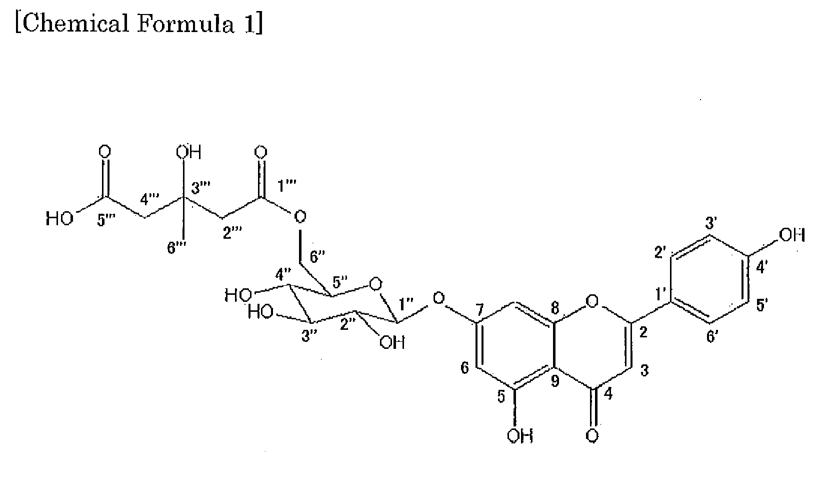

The AP-1 expression inhibitor of the present invention is required only to contain chamaemeloside as described above and neither the composition nor form of the inhibitor is limited. That is, the AP-1 expression inhibitor of the present invention may be composed only of chamaemeloside, only of an extract of Roman chamomile or German chamomile, of a mixture of an extract of Roman chamomile and an extract of German chamomile, only of crushed product of Roman chamomile or German chamomile, of a mixture of crushed product of Roman chamomile and crushed product of German chamomile, or of a mixture thereof, or may contain additional ingredients. The additional ingredients are not limited and can be decided suitably according to, for example, the use of the present invention. Examples thereof include various ingredients and various additives to be described later.

In the present invention, the aforementioned extract of Roman chamomile or German chamomile is not limited and may be, for example, a water extract and a solvent extract, as described below. The solvent extract is preferably an alcohol extract. Examples of the alcohol extract include an isopropyl alcohol extract, an ethanol extract, and a methanol extract. Among these extracts, an ethanol extract is preferable.

The content of chamaemeloside in the expression inhibitor of the present invention is not limited. It also can be decided suitably according to the use of the present invention. Specifically, in the aforementioned expression inhibitor, chamaemeloside is mixed so that the content thereof is, for example, 0.001 to 90 wt%, preferably 0.1 to 90 wt%, in terms of the dry weight. When the expression inhibitor of the present invention is to be formed into, for example, tablets or capsules, the content can be, for example, 0.1 to 90 wt% per tablet or capsule. The expression inhibitor of the present invention of these forms can be commercialized as, for example, pharmaceuticals or supplements to be described below. When the expression inhibitor is to be prepared in the form of a liquid, it can be dissolved in a liquid so that the concentration of the active ingredient chamaemeloside is, for example, 0.01 to 1 wt%, and thereafter a suitable additive is added thereto, which can then be packaged in a bottle container (for example, with a capacity of 100 mL). Examples of the aforementioned liquid include water and alcohol, and examples of the aforementioned additive include a flavor, a sweetener, and a preservative. In order to prevent the content from degenerating, the container is preferably, for example, a brown bottle. The expression inhibitor of the present invention in this form can be commercialized as, for example, health foods and drinks, foods for specified health uses, and supplements. The intake or dose of chamaemeloside is not limited but is preferably 0.01 to 1000 mg/kg and more preferably 0.1 to 100 mg/kg per day per adult. The administration or intake of chamaemeloside may be performed, for example, once daily or a few times a day.

When used, for example, as a skin drug for external use such as an ointment, the expression inhibitor of the present invention is mixed so that the content of chamaemeloside is preferably 0.001 to 10 wt%, and more preferably 0.1 to 1 wt% in, for example, cream, gel, ointment, and cataplasm. When used as, for example, a skin drug for external use such as cosmetics, the expression inhibitor of the present invention is mixed so that the content of chamaemeloside is preferably 0.001 to 10 wt%, and more preferably 0.1 to 1 wt% in, for example, face lotion, milky lotion, cream, and powder.

In the present invention, the raw material for preparation of the aforementioned extract of Roman chamomile or German chamomile, namely the parts of Roman chamomile or German chamomile are not limited. Examples of the raw material include the whole plant body (herb), flower, stem, leaf, branch, branch with leaf, rhizome, root bark, root, or seed of at least one of Roman chamomile and German chamomile. Among these, the whole plant body and flower are preferable. Dried herb or herbal medicine of chamomile, if available, may be used.

The aforementioned extract of Roman chamomile or German chamomile can be obtained by, for example, direct extraction with a solvent added to the aforementioned raw material (parts of Roman chamomile or German chamomile). In addition, it also can be a compressed liquid that is obtained by compression of the aforementioned raw material. Furthermore, for instance, an extract that is obtained by adding a solvent to a residue or a compressed liquid obtained by the compression also can be used.

The aforementioned solvent used for extraction of chamaemelaside is not limited. It can be selected suitably by taking into consideration, for example, the intended use and type of the product to be provided, and subsequent processing and treatments. The aforementioned solvent used for extraction can be, for example, an aqueous solvent, an organic solvent, or a mixture thereof. Specific examples thereof include water; lower alcohols such as methanol, ethanol, propylalcohol, isopropylalcohol, butanol, and isobutanol, or aqueous lower alcohols; polyhydric alcohols such as propylene glycol, 1,3-butyleneglycol, 1,2-butyleneglycol, 1,4-butyleneglycol, 1,5-pentanediol, 1,2-pentanediol, 1,3-pentanediol, 1,4-pentanediol, 1,3,5-pentanetriol, glycerol, and polyethyleneglycol (molecular weight: 100 to 100,000), or aqueous polyhydric alcohols; and various organic solvents such as acetone, ethyl acetate, diethyl ether, dimethyl ether, ethyl methyl ether, dioxane, acetonitrile, xylene, benzene, chloroform, carbon tetrachloride, phenol, and toluene. In addition, examples thereof include acids and alkalis with the normality adjusted suitably. The aforementioned acids can be, for example, hydrochloric acid, sulfuric acid, nitric acid, phosphoric acid, formic acid, and acetic acid, and the aforementioned alkalis can be, for example, sodium hydroxide, potassium hydroxide, calcium hydroxide, and ammonia. With respect to these solvents, any one of them may be used, or two or more of them may be used in combination. For example, only an aqueous solvent such as water may be used depending on the use of the expression inhibitor of the present invention. Furthermore, the following method also may be employed. That is, extraction is performed using a solvent that can be removed easily after the extraction process (for example, a highly-volatile solvent) followed by removal of the solvent, which is then dissolved in the aforementioned aqueous solvent. Moreover, squeeze extraction may be performed suitably.

The aforementioned extraction method is not limited. It can be decided suitably according to, for example, the temperature of the solvent to be used and the ratio in weight of the solvent to the raw material. The extraction time also is not limited and can be set arbitrarily according to, for example, the type of the raw material to be used, the type of the solvent to be used, and the combination thereof. The temperature of the solvent can be set arbitrarily in the range of, for example, -4°C to 100°C. However, it is preferably around 10 to 80°C from the viewpoint of extraction rate and stability of the components contained in the raw material. The ratio in weight of the solvent (B) to the raw material (A) (A:B) also can be set arbitrarily in the range of, for example, 4:1 to 1:50, and the range of 1:1 to 1:20 is particularly preferable.

In the present invention, the aforementioned extract of Roman chamomile or German chamomile further may be subjected to a purification process suitably after extraction as described above. The purification process can be carried out through: decomposition by addition of acid (for example, hydrochloric acid, sulfuric acid, nitric acid, phosphoric acid, or organic acid) or alkali (for example, sodium hydroxide, calcium hydroxide, or ammonia); fermentation or metabolic conversion by microorganisms; adsorption of components to, for instance, ion exchange resin, activated carbon, or diatomite; fractionation by chromatography of various separation modes (for example, ion exchange, hydrophilic adsorption, hydrophobic adsorption, size exclusion, ligand exchange, or affinity); filtration through, for example, filter paper, membrane filter, or ultrafiltration membrane; pressurization or depressurization; heating or cooling; drying; pH adjustment; deodorization or decolorization; and allowing it to stand over a long period of time. With respect to these processes, any one of them can be used or two or more of them can be employed in an arbitrary combination.

In the present invention, the form of the aforementioned extract of Roman chamomile or German chamomile is not limited and is, for example, a liquid, solid, powder, paste, or gel.

The dosage form of the AP-1 expression inhibitor of the present invention is not limited and can be determined arbitrarily according to, for example, the intended use. Specific examples thereof include ampule , capsule, powder, granule, pill, tablet, solid, liquid, gel, bubble, milky lotion, cream, ointment, sheet, mousse, dispersed powder, multi-layer, and aerosol.

The AP-1 expression inhibitor of the present invention is applicable to, for example, pharmaceuticals, quasi-drugs, and other products. The products are not particularly limited and can be, for example, supplements, foods, beverages, food additives, cosmetic materials, and cosmetics.

Pharmaceuticals of the present invention are pharmaceuticals for prevention or treatment of diseases in which a nuclear transcription factor AP-1 is involved or for wound healing, and contain the aforementioned AP-1 expression inhibitor of the present invention. The aforementioned diseases include at least one disease selected from the group consisting of, for example, cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease. The aforementioned skin diseases include at least one disease selected from the group consisting of, for example, acne, Darier's disease, psoriasis, ichthyosis, eczema, sunburn, and atopic dermatitis. The aforementioned malignant hyperproliferative diseases include at least one disease selected from the group consisting of, for example, epithelial cancer, breast cancer, prostate cancer, head and neck cancer, and myeloid leukemia. The aforementioned neointimal hyperproliferative diseases include at least one of, for example, atherosclerosis and restenosis. The aforementioned nonmalignant hyperproliferative diseases include at least one disease selected from the group consisting of, for example, endometrial hyperplasia, benign prostatic hypertrophy, and proliferative vitreoretinopathy. The aforementioned autoimmune diseases include, for example, rheumatoid arthritis. The aforementioned immune diseases include, for example, lupus erythematosus. The aforementioned chronic inflammatory diseases include, for example, pulmonary fibrosis. The aforementioned lipid metabolism/transport-related diseases include, for example, hyperlipidemia. Administration of the pharmaceutical of the present invention can prevent or treat these diseases or heal wounds. The target of administration of the pharmaceutical of the present invention is not limited and can be, for example, humans and mammals other than humans.

The pharmaceutical of the present invention may contain, for example, other AP-1-expression inhibiting ingredients and pharmaceutically allowable additives in addition to the aforementioned expression inhibitor of the present invention. Specific examples of the dosage form of the pharmaceutical of the present invention include tablet, fine granule (including powder), capsule, and liquid (including syrup). Appropriately using, for example, additives and base materials that are suitable for those respective dosage forms, the pharmaceutical of the present invention can be manufactured according to the usual method described in, for example, Japanese Pharmacopoeia. The route of administration is not limited. For example, oral administration and parenteral administration can be employed. Examples of the parenteral administration include intraoral administration, airway administration, intrarectal administration, subcutaneous administration, intramuscular administration, and intravenous administration. The parenteral administration also includes, for example, application to the skin (percutaneous administration).

Supplements of the present invention are supplements for prevention or relief of diseases in which AP-1 is involved or for acceleration of wound healing and contain the aforementioned AP-1 expression inhibitor of the present invention. The aforementioned diseases include at least one disease selected from the group consisting of, for example, cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease. The aforementioned skin diseases include at least one disease selected from the group consisting of, for example, acne, Darier's disease, psoriasis, ichthyosis, eczema, sunburn, and atopic dermatitis. The aforementioned malignant hyperproliferative diseases include at least one disease selected from the group consisting of, for example, epithelial cancer, breast cancer, prostate cancer, head and neck cancer, and myeloid leukemia. The aforementioned neointimal hyperproliferative diseases include at least one of, for example, atherosclerosis and restenosis. The aforementioned nonmalignant hyperproliferative diseases include at least one disease selected from the group consisting of, for example, endometrial hyperplasia, benign prostatic hypertrophy, and proliferative vitreoretinopathy. The aforementioned autoimmune diseases include, for example, rheumatoid arthritis. The aforementioned immune diseases include, for example, lupus erythematosus. The aforementioned chronic inflammatory diseases include, for example, pulmonary fibrosis. The aforementioned lipid metabolism/transport-related diseases include, for example, hyperlipidemia. Administration of the supplement of the present invention can prevent or relieve these diseases and accelerate wound healing. The target of administration of the supplement of the present invention is not limited and can be, for example, humans and mammals other than humans.

The supplement of the present invention may contain, for example, various additives and other supplements in addition to the expression inhibitor of the present invention. Examples of the aforementioned other ingredients include other AP-1-expression inhibiting ingredients, various vitamins such as vitamin C, amino acids, and oligosaccharides. The form of the supplement of the present invention is not particularly limited. Examples thereof include tablet, fine granule (including powder), capsule, and liquid (including syrup).

Foods of the present invention are foods for prevention or relief of diseases in which AP-1 is involved or for acceleration of wound healing and contain the aforementioned AP-1 expression inhibitor of the present invention. The aforementioned diseases include at least one disease selected from the group consisting of, for example, cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease. The aforementioned skin diseases include at least one disease selected from the group consisting of, for example, acne, Darier's disease, psoriasis, ichthyosis, eczema, sunburn, and atopic dermatitis. The aforementioned malignant hyperproliferative diseases include at least one disease selected from the group consisting of, for example, epithelial cancer, breast cancer, prostate cancer, head and neck cancer, and myeloid leukemia. The aforementioned neointimal hyperproliferative diseases include at least one of, for example, atherosclerosis and restenosis. The aforementioned nonmalignant hyperproliferative diseases include at least one disease selected from the group consisting of, for example, endometrial hyperplasia, benign prostatic hypertrophy, and proliferative vitreoretinopathy. The aforementioned autoimmune diseases include, for example, rheumatoid arthritis. The aforementioned immune diseases include, for example, lupus erythematosus. The aforementioned chronic inflammatory diseases include, for example, pulmonary fibrosis. The aforementioned lipid metabolism/transport-related diseases include, for example, hyperlipidemia. Intake of the food of the present invention can prevent or relieve these diseases and accelerate wound healing. Intake of the food of the present invention is not limited but it is taken preferably by, for example, humans and mammals other than humans.

The food of the present invention may contain other various ingredients in addition to the expression inhibitor of the present invention. Examples of the other ingredients include other AP-1-expressian inhibiting ingredients, various additives, and supplements. In the present invention, the foods include, for example, both common foods and functional foods. In the present invention, the form of the food is not limited. Examples thereof include cereals, noodles, confectionery, soups, meats, seafoods, seaweeds, vegetables, fruits, beans, various processed foods, dry foods, frozen foods, smoked foods, canned foods, bottled foods, and retort-packed foods.

Beverages of the present invention are beverages for prevention or relief of diseases in which AP-1 is involved or for acceleration of wound healing and contain the aforementioned AP-1 expression inhibitor of the present invention. The aforementioned diseases include at least one disease selected from the group consisting of, for example, cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease. The aforementioned skin diseases include at least one disease selected from the group consisting of, for example, acne, Darier's disease, psoriasis, ichthyosis, eczema, sunburn, and atopic dermatitis. The aforementioned malignant hyperproliferative diseases include at least one disease selected from the group consisting of, for example, epithelial cancer, breast cancer, prostate cancer, head and neck cancer, and myeloid leukemia. The aforementioned neointimal hyperproliferative diseases include at least one of, for example, atherosclerosis and restenosis. The aforementioned nonmalignant hyperproliferative diseases include at least one disease selected from the group consisting of, for example, endometrial hyperplasia, benign prostatic hypertrophy, and proliferative vitreoretinopathy. The aforementioned autoimmune diseases include, for example, rheumatoid arthritis. The aforementioned immune diseases include, for example, lupus erythematosus. The aforementioned chronic inflammatory diseases include, for example, pulmonary fibrosis. The aforementioned lipid metabolism/transport-related diseases include, for example, hyperlipidemia. Intake of the beverage of the present invention can prevent or relieve these diseases and accelerate wound healing. Intake of the beverage of the present invention is not limited but it is taken preferably by, for example, humans and mammals other than humans.

The beverage of the present invention may contain other various ingredients in addition to the expression inhibitor of the present invention, Examples of the other ingredients include other AP-1-expression inhibiting ingredients, various additives, and supplements. In the present invention, the beverage includes, for example, both common beverages and functional beverages. In the present invention, the form of the beverage is not limited. Examples thereof include soft drinks such as juice, carbonated drink, coffee, black tea, green tea, and mineral water.

Food additives of the present invention are food additives for prevention or relief of diseases in which AP-1 is involved or for acceleration of wound healing and contain the aforementioned AP-1 expression inhibitor of the present invention. The aforementioned diseases include at least one disease selected from the group consisting of, for example, cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease. The aforementioned skin diseases include at least one disease selected from the group consisting of, for example, acne, Darier's disease, psoriasis, ichthyosis, eczema, sunburn, and atopic dermatitis. The aforementioned malignant hyperproliferative diseases include at least one disease selected from the group consisting of, for example, epithelial cancer, breast cancer, prostate cancer, head and neck cancer, and myeloid leukemia. The aforementioned neointimal hyperproliferative diseases include at least one of atherosclerosis and restenosis. The aforementioned nonmalignant hyperproliferative diseases include at least one disease selected from the group consisting of, for example, endometrial hyperplasia, benign prostatic hypertrophy, and proliferative vitreoretinopathy. The aforementioned autoimmune diseases include, for example, rheumatoid arthritis. The aforementioned immune diseases include, for example, lupus erythematosus. The aforementioned chronic inflammatory diseases include, for example, pulmonary fibrosis. The aforementioned lipid metabolism/transport-related diseases include, for example, hyperlipidemia. Intake of the food additive of the present invention can prevent or relieve these diseases and accelerate wound healing. Intake of the food additive of the present invention is not limited but it is taken preferably by, for example, humans and mammals other than humans.

The food additive of the present invention may contain, for example, other various ingredients in addition to the expression inhibitor of the present invention. Examples of the other ingredients include, for example, other AP-1-expression inhibiting ingredients, various additives, and supplements. The form of the food additive of the present invention is not limited. Examples thereof include liquid, powder, flake, and granule. The food additive of the present invention also includes those for beverages in addition to foods.

Cosmetic materials of the present invention are cosmetic materials for prevention or relief of diseases in which AP-1 is involved or for acceleration of wound healing and contain the aforementioned AP-1 expression inhibitor of the present invention. The aforementioned diseases include skin diseases. More specifically, the aforementioned skin diseases include at least one disease selected from the group consisting of, for example, acne, Darier's disease, psoriasis, ichthyosis, eczema, sunburn, and atopic dermatitis. Application of the cosmetic material of the present invention enables prevention or relief of the aforementioned diseases. Application of the cosmetic material of the present invention is not limited but application to, for example, humans and mammals other than humans is preferred.

The cosmetic materials of the present invention may contain, for example, other various ingredients in addition to the expression inhibitor of the present invention. Examples of the other ingredients include other AP-1-expxession inhibiting ingredients and various additives. Examples of the various additives include various fats and oils, waxes, mineral oils, fatty acids, alcohols, polyhydric alcohols, esters, metal soaps, gum, saccharides, water-soluble polymers, surfactants, various vitamins, various amino acids, plant- or animal-derived additives, herbal medicines, oceanic components, metabolites formed by microbial culture, α-hydroxy acids, inorganic pigments, UV absorbers, UV blockers, whitening agents, tyrosinase activity inhibitors, melanin pigment-reducing agents, melanin pigment-decomposing agents, cell activators, astringents, oxygen radical scavengers, antioxidants, lipid peroxide inhibitors, anti-inflammatory agents, antimicrobial agents, germicides, disinfectants, moisturizers, elastase activity inhibitors, agents for hair, antiandrogens, peripheral blood flow-increasing agents, stimulants, metabolic activators, antiseborrheics, keratolytics, oxidizing agents, depilatories, hair-swelling agents, dyes, perfumes, pigments, colorants, sweeteners, nutrient fortifiers, dairy products, hormones, sequestering agents, pH adjusters, chelating agents, antiseptics, antifungal agents, fresheners, stabilizers, emulsifiers, blood flow-increasing agents, anti-inflammatory agents, antiphlogistics, antiallergic agents, wound therapeutics, bubble promoters, thickeners, enzymes, purified water, agents for oral cavity, deodorizers, deodorants, bitters, and seasonings.

Cosmetics of the present invention contain the aforementioned cosmetic materials of the present invention. Application of the cosmetics of the present invention enables prevention or relief of the aforementioned skin diseases or acceleration of wound healing. The cosmetics of the present invention are applied preferably to, for example, humans and mammals other than humans. The cosmetics of the present invention may contain, for example, other additives in addition to the cosmetic materials of the present invention. For example, the additives can be those described with respect to the aforementioned cosmetic materials. The form of the cosmetic of the present invention is not particularly limited. Examples thereof include basic cosmetics such as face lotion, milk lotion, cream, ointment, lotion, oil, and pack; skin cleansers such as soap, cleansing cream, cleansing lotion, and facial wash; cosmetics for hair washing such as shampoos, conditioners, and treatments; hairdressings such as hair cream, hair spray, hair tonic, hair gel, hair lotion, hair oil, hair essence, hair water, hair wax, and hair foam; hair restorer and hair growth tonic; hair dyes such as single-component hair dyes, two-component hair dyes, and hair colors; hair cosmetics such as perm solutions including permanent wave lotions and hair relaxers, and hair cosmetics such as permanent wave keeping agents; makeup cosmetics such as foundation, powder, face powder, lipstick, cheek rouge, eye shadow, eye liner, mascara, eyebrow pencil, and false eyelashes; finishing cosmetics such as manicures; and cosmetic compositions such as perfumes. Other examples include tooth pastes; compositions for oral cavity such as gargle; formulations for external medicine; ointments; cataplasm; bath agents; medicinal compositions for oral cavity such as medicated toothpastes and mouth fresheners; medical cosmetics; solvents for hair such as solvents for permanent wave solutions, hair dyes, hair restorers, hair loss inhibitors, and depilatories; agents for preventing underarm odor and body odor; and quasi-drugs for external use such as sanitary goods, sanitary cottons, and wet tissues.

The present invention provides a method of inhibiting expression of AP-1 and the method includes a step of administering the aforementioned chamaemeloside. Administration of chamaemeloside can inhibit the expression of AP-1 in cells. The target of administration of chamaemeloside is not limited and may be, for example, a biological body, cells isolated from a biological body, or cultured cells. The aforementioned biological body is not limited. Examples thereof include humans and mammals including humans. The form of the aforementioned chamaemeloside to be administered is not limited. Examples thereof include purified chamaemeloside or the extract or crushed product of the whole plant body or a part of Roman chamomile or German chamomile as described above. Specific examples of the form of chamaemeloside in the present invention include the same forms as those of the AP-1 expression inhibitor, pharmaceuticals, and various products of the present invention described above.

The aforementioned method of administration is not limited. Examples thereof include oral administration and parenteral administration in the case of a biological body. Examples of the parenteral administration include intraoral administration, airway administration, intrarectal administration, subcutaneous administration, intramuscular administration, intravenous administration, and application to skin (percutaneous administration). In the case of the cells isolated from a biological body, there is a method in which they are cultured in a medium with chamaemeloside added thereto.

The present invention is a method for preventing or treating diseases in which AP-1 is involved or for healing wounds and includes a step of administering chamaemeloside. The form of the AP-1 expression inhibitor of the present invention is not limited and the same as described above. The AP-1 expression inhibitor may be administered, for example, as an inflammation relieving agent, pharmaceutical, supplement, food, beverage, or food additive as described above.

The aforementioned administration method is not limited. Examples thereof include oral administration and parenteral administration. Examples of the parenteral administration include intraoral administration, airway administration, intrarectal administration, subcutaneous administration, intramuscular administration, intravenous administration, and application to skin (percutaneous administration). The target of administration of chamaemeloside is not limited and for example, humans or mammals including humans.

Next, the examples of the present invention are described. The present invention is not limited to the following examples.

### [Example 1]

This example is an example in which expression inhibition of a nuclear transcription factor AP-1 at the gene level by chamaemeloside was confirmed.

### (1) Preparation of extract of Roman chamomile

One kilogram of capitula of dried Roman chamomile (*Anthemis nobilis* Linn.) herb was immersed in 20 kg of 50% (v/v) ethanol for 1 week and thereby an extraction treatment was carried out. The extraction treatment was followed by suction filtration, and thereby 12.63 kg of Roman chamomile extract was obtained.

### (2) Purification of chamaemeloside

Chamaemeloside was purified by the following procedure. The aforementioned Roman chamomile extract was freeze-dried. Then 10.31 g of this dried material (the solid content of the extract) was suspended in 750 mL of water (H₂O adjusted to pH 4, followed by partition using chloroform. The chloroform layer was removed and further ethyl acetate was added to the aqueous layer, which was subjected to partition. Thus 2.80 g of ethyl acetate layer (extract partitioned to ethyl acetate) was obtained. The pH 4 extract partitioned to ethyl acetate was dissolved in 50 mL of methanol, to which silica gel was added. This allowed the silica gel to adsorb the aforementioned Roman chamomile extract contained in the ethyl acetate layer. The mixture was stirred with a spatula on a heating bath and was then concentrated under reduced pressure, so that the organic solvents were removed completely. The resulting concentrate is referred to as a silica gel-containing concentrated fraction.

The silica gel-containing concentrated fraction was subjected to the first purification with the column chromatography described below. The aforementioned silica gel-containing concentrated fraction was suspended in a mixture of chloroform and methanol (volume ratio: 8/1) and the pH thereof was adjusted to 4. A chromatography tube with a diameter of 5 cm and a height of 20 cm was filled with about 400 cm³ of silica gel that had been equilibrated with a mixture of chloroform and methanol (volume ratio: 7/1) and then was filled with the aforementioned suspension of the silica gel-containing concentrated fraction on top thereof. Then elution was performed using about 1200 mL each of the mixtures of chloroform and methanol (volume ratios: 7/1, 5/1, 3/1, and 1/1) and methanol in this order. The fraction eluted with the mixture of chloroform and methanol (volume ratio: 1/1) was collected and the solvent contained therein was removed under reduced pressure.

Then the fraction collected by the first column chromatography was subjected further to the second purification with the following column chromatography. The collected fraction that had been concentrated was dissolved in a mixture of acetonitrile-water-TFA (volume ratio: 10/90/0.1). Separately, a chromatography tube with a diameter of 3 cm was filled with about 60 cm³ of a carrier (trade name: Cosmosil140 C18-OPN) suspended in methanol and equilibrated with 600 mL of a mixture of acetonitrile-water-TFA (volume ratio: 10/90/0.1). To this chromatography tube, the aforementioned collected fraction dissolved in the mixture of acetonitrile-water-TFAwas applied. Then elution was performed using 180 mL each of mixtures of acetonitrile-water-TFA (volume ratios: 10/90/0.1, 18/82/0.1, and 25/75/0.1) and acetonitrile in this order. The fraction eluted with the mixture of acetonitrile-water-TFA (volume ratio: 25/75/0.1) was collected.

The resultant collected fraction was concentrated under reduced pressure and freeze-dried. The freeze-dried product was redissolved in methanol, and this methanol solution was subjected to preparative HPLC under the following conditions, so that chamaemeloside was isolated. In the following conditions, TFA refers to trifluoroacetic acid. The sample was a methanol solution containing about 100 mg/mL of the solid content of Roman chamomile extract.

### HPLC conditions

Column: COSMOSIL 5C18-MS (trade name) 250 × 10 mm I.D.
Eluent: Acetonitrile-water-TFA (volume ratio: 20/80/0.1)
Flow rate: 4.7 mL/min
Detection: UV at 220 nm
Column temperature: 40°C
Quantity of sample injected: 50 µL
Collection time: 14 to 16 min

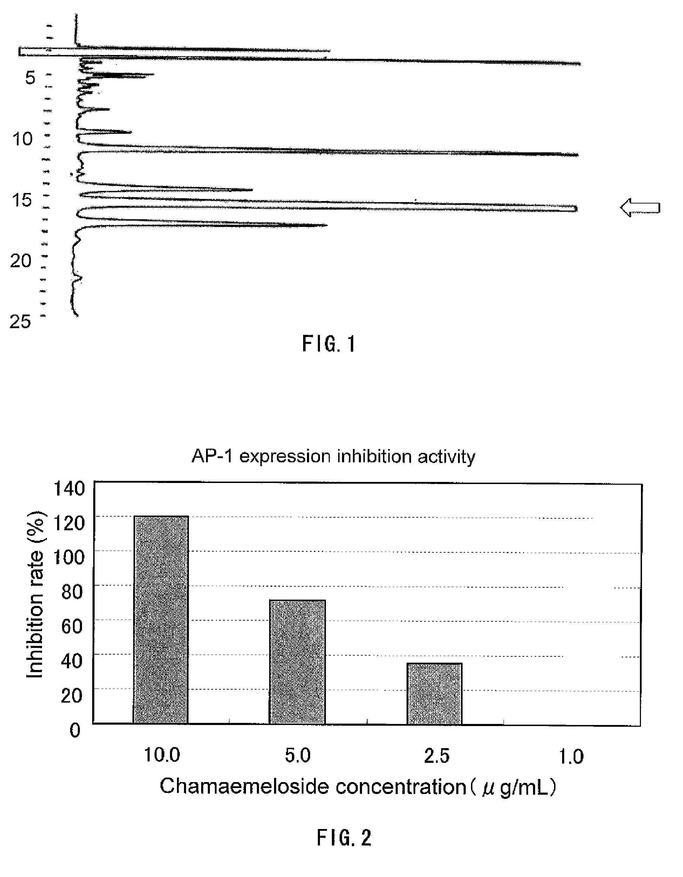

An HPLC chromatogram is shown in Fig.1. In FTG.1, the peak represented by the arrow is the peak of chamaemeloside. The chamaemeloside was identified as follows. That is, the material isolated by HPLC was subjected to nuclear magnetic resonance spectral (NMR) measurement, and then it was checked whether the measurement value was in agreement with the analytical value described in the reference (Gabriela et al., Phytochemistry, 41:643-646, 1996). The aforementioned NMR data are shown in Tale 1 below together with the data described in the aforementioned reference. As a result, 32 mg of purified chamaemeloside was obtained from 300 mg of Roman chamomile extract.

**[Table 1]**

| Results of NMR measurement of Roman chamomile extract | | | | | | | | | | | |
|---|---|---|---|---|---|---|---|---|---|---|---|
| Identification results | ¹³C-NMR | ¹H-NMRIDMSO-d₆+D₂O | | | | ¹H-NMR/DMSO-d₆ | | | | Reference Data¹⁾ | |
| (C structure) | ppm | | ppm | | | | ppm | | | ¹³C-NMR(ppm) | ¹H-NMR(ppm) |
| 2 | 164.4 | | | | | | | | | 164.5 | |
| 3 | 103.3 | 6.84 | | | | 6.85 | | | | 103.3 | 6.87 |
| 4 | 182.1 | | | | | | | | | 182.2 | |
| 5 | 160.9 | | | | | | | | | 161.3 | |
| 6 | 99.6 | 6.49 | 6.47 | | | 6.48 | 6.48 | | | 99.7 | 6.43 |
| 7 | 162.7 | | | | | | | | | 162.7 | |
| 8 | 94.9 | 6.83 | | | | 6.84 | | | | 94.9 | 6.80 |
| 9 | 157.1 | | | | | | | | | 157.1 | |
| 10 | 105.5 | | | | | | | | | 105.6 | |
| 1' | 121.2 | | | | | | | | | 121.2 | |
| 2' | 128.8 | 7.99 | 8.01 | | | 7.99 | 8.01 | | | 128.8 | 7.96 |
| 3' | 116.1 | 6.91 | | | | 6.90 | | | | 116.2 | 6.94 |
| 4' | 116.1 | | | | | | | | | 116.5 | |
| 5' | 116.1 | 6.97 | 6.99 | | | 6.98 | 7.00 | | | 116.2 | 6.94 |
| 6' | 128.8 | 7.99 | | | | 7.99 | | | | 128.8 | 7.96 |
| 1" | 99.6 | 5.15 | 5.16 | | | 5.15 | 5.16 | | | 99.7 | 5.12 |
| 2" | 72.9 | 3.31 | 3.32 | | | 3.30 | 3.32 | 3.33 | | 73.1 | 3.31 |
| 2"(OH) | | 5.53 | 5.54 | | | | | | | | 5.50 |
| 3" | 76.0 | 3.36 | 3.38 | | | 3.35 | 3.36 | 3.38 | | 76.3 | 3.36 |
| 3"(OH) | | 5.30 | | | | | | | | | 5.28 |
| 4" | 69.7 | 3.20 | 3.22 | 3.24 | | 3.20 | 3.21 | 3.22 | 3.24 | 69.9 | 3.19 |
| 4"(OH) | | 5.39 | | | | | | | | | 5.35 |
| 5" | 73.9 | 3.76 | 3.78 | 3.79 | | 3.77 | 3.78 | 3.80 | | 74.1 | 3.74 |
| 6"(A) | 63.3 | 4.35 | 4.37 | | | 4.36 | 4.38 | | | 63.4 | 4.33 |
| 6"(B) | | 4.09 | 4.10 | 4.11 | 4.13 | 4.10 | 4.11 | 4.12 | 4.13 | | 4.08 |
| 1"' | 170.6 | 2.61 | 2.65 | 2.68 | | 2.61 | 2.65 | 2.68 | | 170.6 | 2.64 |
| 2"' | 45.3 | 2.53 | 2.54 | 2.54 | 2.58 | 2.52 | 2.54 | 2.54 | 2.58 | 45.4 | 2.55 |
| 3'" | 68.9 | 2.46 | 2.49 | | | 2.46 | 2.49 | | | 69.0 | 2.48 |
| 4"' | 45.0 | 2.40 | 2.43 | | | 2.41 | 2.43 | | | 45.2 | 2.40 |
| 5"' | 172.4 | | | | | | | | | 172.5 | |
| 6"' | 27.5 | 1.21 | | | | 1.21 | | | | 27.6 | 1.18 |

| | | | | | | | | | | | |
|---|---|---|---|---|---|---|---|---|---|---|---|
| 1) Gabriela et al: Phytochemistry, 41(2): 643-646, 1996 | | | | | | | | | | | |

### (3) Measurement of activity of nuclear transcription factor AP-1

HeLa cells were tripsinized and centrifuged at 4°C and 1,000 rpm for 3 minutes. The collected Hela cells were seeded on a 30-mm dish so that the density was 2 × 10⁵ cells/well, which served as a test plate. With the test plate being placed at 37°C for 12 hours in the presence of 5% CO₂ the cells were cultured and were then transformed. For the transformation, two types of vectors were transfected into the aforementioned cells using a transformation reagent (trade name: Effectene Transfection Reagent, QIAGEN). The vectors used herein were pAP-1 (PMA)-SEAP (trade name) (CLONTECH) (0.4 µg) and pLuc"control (trade name) (CLONTECH) (0.02 µg). The cells transfected with both of the vectors were cultured at 37°C for 16 hours in the presence of 5% CO₂ and then the cultured cells were detached from the aforementioned test plate using trypsin. The detached cells were dispensed into a 96-well plate so that 0.2 × 10⁴ cells were contained in each well. The cells thus dispensed were cultured for two hours. Thereafter, the aforementioned chamaemeloside solution was added thereto so that the final concentrations (the weight of the solid content of the extract) were 10 µg/mL, 5.0 µg/mL, 2.5 µg/mL, and 1.0 µg/mL, and further TPA (12-O-tetradecanoylphorbol acetate) was added thereto so that the final concentration was 4 µg/mL. Twenty-four hours after the addition, the medium was collected in 25-µL portions. The medium thus collected was evaluated for the secreated alkaline phosphatase (SEAP) activity based on the activity of the nuclear transcription factor AP-1 contained in the medium, using a kit (trade name: Great EscAPe SEAP kit, CLONTECK). The gene expression was corrected using the luciferase activity value obtained by measurement carried out using the luciferase activity measuring kit (Promega). The results are shown in the graph in FIG.2. The evaluation for inhibition of the nuclear transcription factor AP-1 expression was indicated by a relative value in % (inhibition rate in %) of secreated alkaline phosphatase (SEAP) activity, with the luciferase activity at each concentration of chamaemeloside that had been added being considered as 100%.

As shown in the graph in FIG.2, addition of chamaemeloside allowed expression of the nuclear transcription factor AP-1 to be suppressed (inhibited) in a concentration-dependent manner. The IC₅₀ of chamaemeloside was 5 µg/mL.

### (4) Chamaemeloside in German chamomile

The extract of German chamomile was prepared and the extract was subjected to the HPLC analysis under the aforementioned conditions. Thereby the presence of chamaemeloside was confirmed.

Two grams of capitula of dried German chamomile *(Maticaia recutita)* herb was immersed in 200 mL of distilled water at 80°C for 10 minutes and thereby an extraction treatment was carried out. The extraction treatment was followed by suction filtration, and thereby 200 mL of German chamomile extract was obtained. The extract was subjected to HPLC under the conditions described below. In addition, the aforementioned chamaemeloside (32 µg/ml) purified by HPLC from Roman chamomile also was subjected to HPLC under the same conditions.

### HPLC conditions

Column COSMOSIL 5C₁₈-AR-II (trade name) 250 × 4.6 mm LD.
Eluent: Acetonitrile-water-TFA (volume ratio: 20/70/0.1)
Flow rate: 1.0 mL/min
Detection: UV at 340 nm
Column temperature: 40°C
Quantity of sample injected: 20 µL

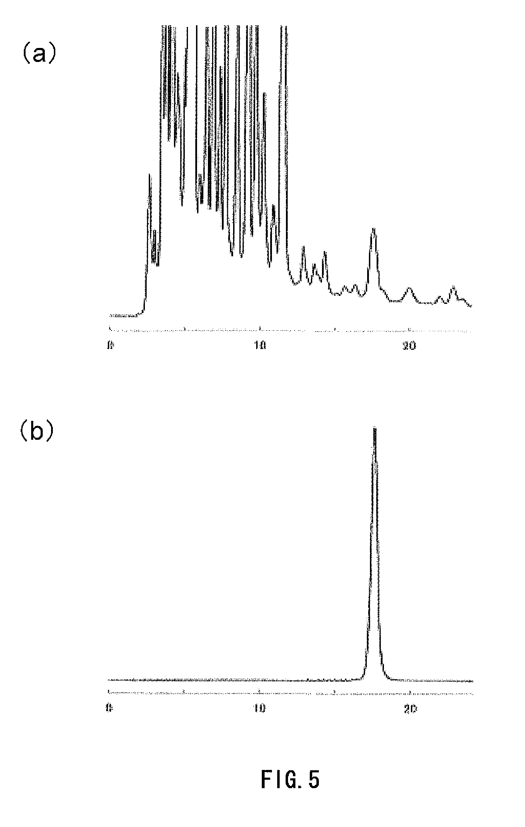

The chromatograms thus obtained are shown in FIG.5. In FIG.5, (a) is the chromatogram of the German chamomile extract and (b) is the chromatogram of chamaemeloside derived from Roman chamomile. As shown in (a), it was confirmed that the German chamomile extract had a peak at the same retention time as that of the Roman chamomile-derived chamaemeloside shown in (b). From this result, it was identified that chamaemeloside was contained in the German chamomile extract. As described above, German chamomile also contains chamaemeloside and therefore it is evident that chamaemeloside of the present invention also can be prepared using German chamomile as the raw material, as in the case of Roman chamomile, and the AP-1 expression-inhibiting effect can be obtained.

### <Reference Example>

This Reference Example is an example in which inhibition of NF-κB activity was evaluated using the Roman chamomile extract and chamaemeloside obtained in Example 1.

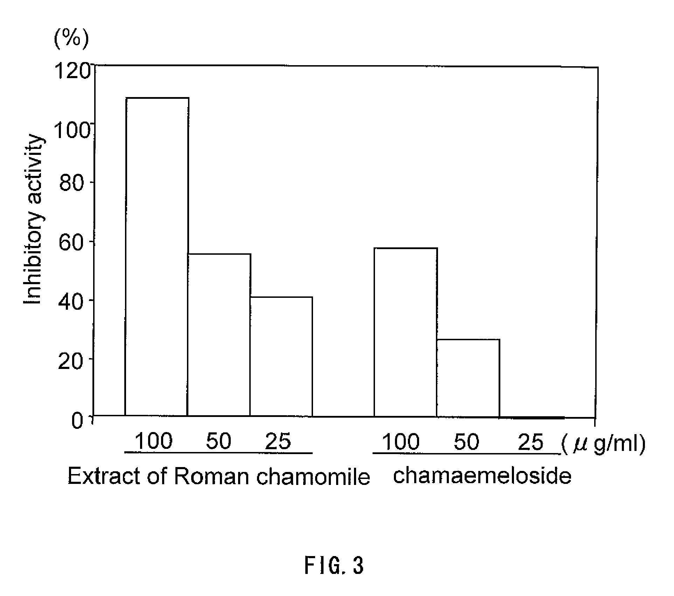

HeLa cells were tripsinized and centrifuged at 4°C and 1,000 rpm for 3 minutes. The collected Hela cells were seeded on a 30-mm dish so that the density was 2 × 10⁵ cells/well, which served as a test plate. With the test plate being placed at 37°C for 12 hours in the presence of 5% CO₂, the cells were cultured and were then transformed. For the transformation, two types of vectors were transfected into the aforementioned cells using a transformation reagent (trade name: Effectene Transfection Reagent, QIAGEN). The vectors used herein were pNF-κB-SEAP (trade name) (CLONTECH) (0.4 µg) and pLuc-control (trade name) (CLONTECH) (0.02 µg). The cells transfected with both of the vectors were cultured at 37°C for 16 hours in the presence of 5% CO₂ and then the cultured cells were detached from the aforementioned test plate using trypsin. The detached cells were dispensed into a 96-well plate so that 0.2 × 10⁴ cells were contained in each well. The cells thus dispensed were cultured for two hours. Thereafter, the aforementioned Roman chamomile extract was added thereto so that the final concentrations (the weight of the solid content of the extract) were 100 µg/mL, 50 µg/mL, and 25 µg/mL, and further TNF (tumor necrosis factor)-alpha was added thereto so that the final concentration was 40 ng/mL. Twenty-flour hours after the addition, the medium was collected in 25-µL portions. The medium thus collected was evaluated for the secreated alkaline phosphatase (SEAP) activity based on the activity of NF"κB contained in the medium using a kit (trade name: Great EscAPe SEAP kit, CLONTECK). The gene expression was corrected using the luciferase activity value obtained by measurement carried out using the luciferase activity measuring kit (Promega). The results are shown in the graph in FIG.3. The evaluation for inhibition of NF-κB expression was indicated by a relative value in % (inhibition rate in %) of secreated alkaline phosphatase (SEAP) activity, with the luciferase activity at each concentration of the substance to be tested (Roman chamomile extract or chamaemeloside solution) that had been added being considered as 100%.

As shown in the graph in FIG.3, both the Roman chamomile extract and chamaemeloside inhibited the expression of NF-κB in a concentration-dependent manner. The IC₅₀ of the Roman chamomile extract was 50 µg/mL and that of chamaemeloside was 80 µg/mL.

### Industrial Applicability

As described above, the expression inhibitor of a nuclear transcription factor AP-1 of the present invention is characterized by containing chamaemeloside, is excellent in safety, and causes no problems even after intake over a prolonged period of time. Furthermore, the expression inhibitor of the present invention provides an excellent activity of inhibiting the expression of a nuclear transcription factor AP-1. Therefore the expression inhibitor of a nuclear transcription factor AP-1 of the present invention is applicable to various goods such as pharmaceuticals, quasi-drugs, supplements, foods, beverages, and food additives, and the use thereof is wide ranging and not limited.

## Claims

1. An expression inhibitor of a nuclear transcription factor AP-1, comprising chamaemeloside.

2. The expression inhibitor of AP-1 according to claim 1, wherein expression inhibition of the AP-1 is at least one of expression inhibition at a gene level and expression inhibition at a protein level.

3. The expression inhibitor of a nuclear transcription factor AP-1 according to claim 1, wherein the chamaemeloside is at least one of chamaemeloside derived from Roman chamomile and chamaemeloside derived from German chamomile.

4. The expression inhibitor of a nuclear transcription factor AP-1 according to claim 1, comprising at least one of an extract of Roman chamomile and an extract of German chamomile.

5. The expression inhibitor of a nuclear transcription factorAP-1 according to claim 4, wherein the extract is at least one selected from the group consisting of an alcohol extract and a water extract of Roman chamomile and an alcohol extract and a water extract of German chamomile.

6. A pharmaceutical for prevention or treatment of a disease in which a nuclear transcription factor AP-1 is involved or for wound healing,
wherein the pharmaceutical comprises an expression inhibitor of a nuclear transcription factor AP-1 according to claim 1.

7. The pharmaceutical according to claim 6, wherein the disease is at least one selected from the group consisting of cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease.

8. The pharmaceutical according to claim 7, wherein the skin diseases include at least one selected from the group consisting of acne, Darier's disease, psoriasis, ichthyosis, eczema, sunburn, and atopic dermatitis,
the malignant hyperproliferative diseases include at least one selected from the group consisting of epithelial cancer, breast cancer, prostate cancer, head and neck cancer, and myeloid leukemia,
the neointimal hyperproliferative diseases include at least one of atherosclerosis and restenosis,
the nonmalignant hyperproliferative diseases include at least one selected from the group consisting of endometrial hyperplasia, benign prostatic hypertrophy, and proliferative vitreoretinopathy,
the autoimmune diseases include rheumatoid arthritis,
the immune diseases include lupus erythematosus,
the chronic inflammatory diseases include pulmonary fibrosis, and
the lipid metabolism/transport-related diseases include hyperlipidemia.

9. A product for prevention or relief of a disease in which a nuclear transcription factor AP-1 is involved or for acceleration of wound healing,
wherein the product comprises an expression inhibitor of a nuclear transcription factor AP-1 according to claim 1.

10. The product according to claim 9, wherein the disease is at least one selected from the group consisting of cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease.

11. The product according to claim 10, wherein the skin diseases include at least one selected from the group consisting of acne, Darier's disease, psoriasis, ichthyosis, eczema, sunburn, and atopic dermatitis,
the malignant hyperproliferative diseases include at least one selected from the group consisting of epithelial cancer, breast cancer, prostate cancer, head and neck cancer, and myeloid leukemia,
the neointimal hyperproliferative diseases include at least one of atherosclerosis and restenosis,
the nonmalignant hyperproliferative diseases include at least one selected from the group consisting of endometrial hyperplasia, benign prostatic hypertrophy, and proliferative vitreoretinopathy,
the autoimmune diseases include rheumatoid arthritis,
the immune diseases include lupus erythematosus,
the chronic inflammatory diseases include pulmonary fibrosis, and
the lipid metabolism/transport-related diseases include hyperlipidemia.

12. The product according to claim 9, wherein the product is a supplement, food, beverage, food additive, cosmetic material, or cosmetic.

13. A method of inhibiting expression of a nuclear transcription factor AP-1, wherein the method comprises administering chamaemeloside.

14. A method for preventing or treating a disease in which a nuclear transcription factor AP-1 is involved or for healing a wound, wherein the method comprises administering chamaemeloside.

15. The method for preventing or treating a disease or for healing a wound according to claim 14, wherein the disease is at least one selected from the group consisting of cancer, metastasis of cancer, arteriosclerosis, hypertension, diabetes, skin diseases, malignant hyperproliferative diseases, neointimal hyperproliferative diseases, nonmalignant hyperproliferative diseases, autoimmune diseases, immune diseases, arthritis, asthma, allergy, chronic inflammatory diseases, lipid metabolism/transport-related diseases, dry-eye syndrome, neurodegenerative diseases, Alzheimer-type diseases, and Parkinson disease.

16. The method for preventing or treating a diseases or for healing a wound according to claim 15, wherein the skin diseases include at least one selected from the group consisting of acne, Darier's disease, psoriasis, ichthyosis, eczema, sunburn, and atopic dermatitis,
the malignant hyperproliferative diseases include at least one selected from the group consisting of epithelial cancer, breast cancer, prostate cancer, head and neck cancer, and myeloid leukemia,
the neointimal hyperproliferative diseases include at least one of atherosclerosis and restenosis,
the nonmalignant hyperproliferative diseases include at least one selected from the group consisting of endometrial hyperplasia, benign prostatic hypertrophy, and proliferative vitreoretinopathy,
the autoimmune diseases include rheumatoid arthritis,
the immune diseases include lupus erythematosus,
the chronic inflammatory diseases include pulmonary fibrosis, and
the lipid metabolism/transport-related diseases include hyperlipidemia.

17. Use of chamaemeloside for inhibiting expression of a nuclear transcription factor AP-1.

18. Use of chamaemeloside for preventing or treating a disease in which a nuclear transcription factor AP-1 is involved or for healing a wound.
